# EUROPEAN PATENT SPECIFICATION

(11) **EP 3 988 037 B1**
(45) Date of publication and mention of the grant of the patent: **24.01.2024**
(21) Application number: 20306248.4
(22) Date of filing: 20.10.2020
(51) Int. Cl.: A61B 17/16

(54) **TOOL HOLDER FOR MODULAR TOOL**
WERKZEUGHALTER FÜR MODULARES WERKZEUG
PORTE-OUTIL POUR OUTIL MODULAIRE

(43) Date of publication of application: 27.04.2022
(73) Proprietor: Ostium Group, 44360 Saint-Etienne de Montluc (FR)
(72) Inventor: BIBARD, Léopold, 44360 Saint-Etienne de Montluc (FR); LE HENANF, Corentin, 44360 Saint-Etienne de Montluc (FR); RETAILLEAU, Vincent, 44360 Saint-Etienne de Montluc (FR); SOQUENNE, Edgard, 44360 Saint-Etienne de Montluc (FR)
(74) Representative: Atout PI Laplace

(56) References cited:
- WO-A1-2011/073632
- IT-A1- UD20 100 110
- US-A1- 2007 167 952

## Description

### Field of the invention

The present invention relates to modular tools, and in particular a tool holder element for such tools.

### Background of the invention

The concept of modularity in tools is almost as old as tool use itself. Many ancient tool designs such as hammers, axes, picks and the like comprise a working element fixed to a handle by some means which allows the periodic replacement of one element or the other. Developments in manufacturing processes over the last two hundred years meanwhile have made it possible to develop releasable fixing mechanisms. For example, screw driver heads are commonly available as 6.35 mm (quarter inch) hexagon bits, which may be inserted as required in a standard handle. The bits may be secured magnetically, or by means of a spring loaded ball bearing engaging a groove in the bit. In some contexts meanwhile, the operational constraints extant in that context may tend to lead away from such approaches. In the field of surgical instruments for example, the high forces involved along with sterilisation requirements may tend to indicate an all-in-one approach.

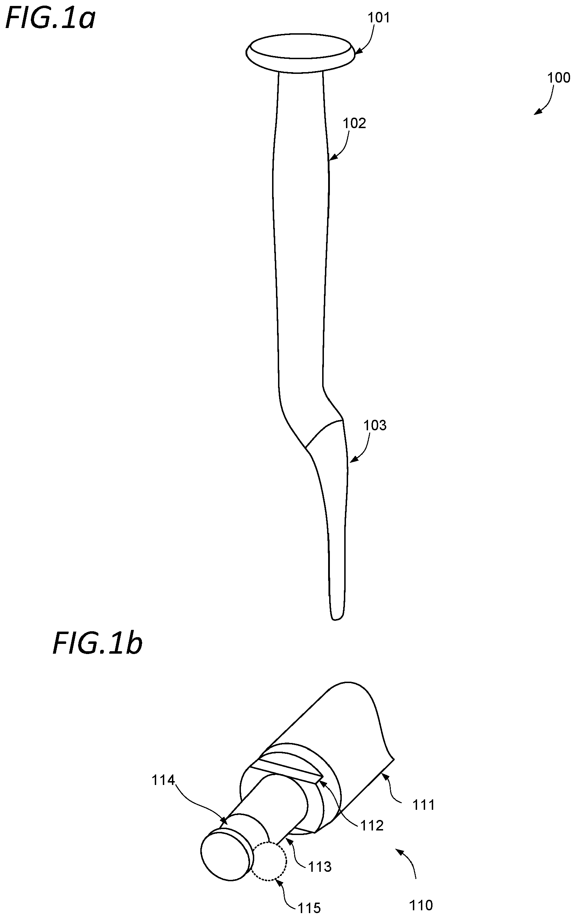

Figure 1a shows an orthopaedic reamer as known in the state of the art.

As shown in figure 1a, a reamer 100 comprises a handle 102 with a strike head 101 and a reamer working surface 103. The reamer 100 is typically made entirely of stainless steel.

Notwithstanding the foregoing, the cost of manufacturing a complete set of stand-alone tools for example as shown in figure 1 can be considerable, and even in the field of surgical tools, some attempts at modular tools are known.

In this, as in any field where a solid positive engagement between the handle and further elements is a critical requirement, special consideration must be given to the securing mechanism.

Figure 1b shows a releasable securing mechanism for modular tools as known in the state of the art.

Figure 1b shows a conventional securing mechanism known in the art as a "Hudson Fitting". In particular, figure 1b shows a male Hudson fitting 110, attached to a tool element 111. The fitting comprises a cylindrical member 113 with a semi-circular channel or groove 114. When the cylindrical member 113 is slid into the corresponding female element, a spring loaded ball bearing 115 engages the channel so as to prevent accidental decoupling. In implementations where uncoupling must be avoided in the presence of a separating force, the ball bearing may be replaced with a removable cotter pin or the like. As shown, the fitting also comprises a flattened flange 112 at the proximal end of the fitting closest to the tool element 111. The flats of this flange may engage corresponding surfaces on the corresponding female element when the coupling is fully inserted, so that rotational forces may be effectively transferred between the two elements of the coupling.

US2007/0167952 describes a surgical tool holder intended to aid a surgeon in controlling the use of a tool during, for example, preparation of a femoral cavity for reception of hip joint prosthesis. The holder has a housing that encloses a mechanism having, at a far end, a tool-engaging interface, and at the opposite end, a handle which facilitates manipulation of the tool during use in preparing a bone site by the surgeon. The holder enables orientation of the tool attached to its end, which is important because control of the tool is critical in order to accurately prepare a recess for reception and installation of a prosthesis.

Fittings such as that shown in figure 1b have been found unsatisfactory in terms of their ability to provide a solid positive engagement between the handle and further elements, whilst supporting uncoupling with a minimum of force and dexterity when required, yet averting the risk of accidental uncoupling. As such, it is desired to provide an improved coupling addressing some or all of these concerns.

### Summary of the invention

According to the present invention in a first aspect there is provided a modular tool comprising a tool holder. The tool holder is provided with an entry aperture, and adapted to receive a proximal extremity of a tool element in said entry aperture. The modular tool further comprises a securing pin assembly comprising a securing pin, and a securing pin channel extending though said tool holder and terminating in the entry aperture. The securing pin is situated in the securing channel so as to slidingly move between a first, retracted position and a second, extended position in which the securing pin protrudes into the entry aperture, whereby in the second position the securing pin traps the proximal extremity of said tool element in the entry aperture, the proximal extremity being unconstrained by the securing pin in the first position. The modular tool further comprises a rocker element having a fulcrum pivotally coupled to the tool holder, a first arm on one side of the fulcrum pivotally coupled to the securing pin, and an eccentric arm on the opposite side of the fulcrum to the first arm, such that either
a. pressure on the first arm biases the securing pin towards the first position, and pressure on the second arm biases the securing pin towards the second position, or
b. pressure on the first arm biases the securing pin towards the second position, and pressure on the second arm biases the securing pin towards the first position.

In a development of the first aspect, the arm upon which pressure biases the securing pin towards the first position, and the tool holder, are provided with a resiliently biased detent such that when sufficient pressure is exerted on the arm, the retention mechanism engages to releasably lock the arm in a first predetermined position, and thereby maintain the securing pin in the first position.

In a further development of the first aspect, the arm upon which pressure biases the securing pin towards the second position, and the tool holder, are provided with a resiliently biased detent such that when sufficient pressure is exerted on the arm, the retention mechanism engages to releasably lock the arm in a second predetermined position, and thereby maintain the securing pin in the second position.

In a further development of the first aspect, the resiliently biased detent comprises a depression formed in the tool holder and a corresponding rounded pin on a resilient projection from the rocker.

In a further development of the first aspect, the resiliently biased detent comprises a first depression formed on one side of the tool holder and a second depression formed on one the of said tool holder and a corresponding first rounded pin on a respective first resilient projection from one side of the rocker and a corresponding second rounded pin on a respective second resilient projection from the opposite side of the rocker, so that the first and second rounded pins are biased towards each other when engaged with said respective first and second depressions.

In a further development of the first aspect, the fulcrum is defined by a further resilient projection from each side of the rocker, each further resilient projection being provided with a fulcrum pin, whereby the fulcrum pin on each further resilient projection is situated on an axis defining the axis of said fulcrum, the tool holder being provided with apertures engaging the fulcrum pins.

In a further development of the first aspect, the first arm is pivotally coupled to the securing pin by means of a fork or hook disposed on the securing pin releasably engaging a pivot pin disposed in the rocker parallel the axis of the fulcrum.

In a further development of the first aspect, fork or hook being formed of a resilient material and so dimensions as to be resiliently retained on said pivot pin.

In a further development of the first aspect, the tool holder having a distal end and a proximal end disposed on a longitudinal axis, wherein the entry aperture comprises a first lateral slot disposed along a second axis in the plane of the first axis and being at an angle of between 10 and 80 degrees to the longitudinal axis, the slot widening from top to bottom, said first slot widening to an entry aperture at the distal end of the first slot, the a securing pin channel extending though the tool holder.

In a further development of the first aspect, the dimensions of the lateral slot vary along the second axis so that the force required to slide a corresponding keyed element of said tool into said lateral slot increases as the corresponding keyed element of said tool progresses into the slot.

In a further development of the first aspect, the tool holder comprised a second slot parallel to the first slot.

In a further development of the first aspect, the second slot is on the same axis as the first slot, and positioned on the distal side thereof.

In a further development of the first aspect, the second slot provides a lateral opening in said tool holder.

In a further development of the first aspect, the tool holder is composed of a synthetic material or a synthetic composite material.

In a further development of the first aspect, the tool holder is composed of a glass fiber reinforced polyarylamide.

### Brief Description of the Drawings

The above and other advantages of the present invention will now be described with reference to the accompanying drawings, for illustration purposes only, in which:
- Figure 1a: shows an orthopaedic reamer as known in the state of the art;
- Figure 1b: shows a releasable securing mechanism for modular tools as known in the state of the art;
- Figure 2a: shows a side view of a modular tool in accordance with an embodiment in a first configuration;
- Figure 2b: shows a side view of a modular tool in accordance with the embodiment of figure 2a in a second configuration;
- Figure 3: shows a side view of a modular tool in accordance with a further embodiment;
- Figure 4: shows a three-dimensional view of a rocker in accordance with an embodiment;
- Figure 5: shows the rocker of figure 4 mounted on a tool holder in accordance with an embodiment;
- Figure 6: shows a tool holder for a modular tool in accordance with an embodiment in a first configuration;
- Figure 7: shows a tool holder for a modular tool in accordance with an embodiment in a second configuration;
- Figure 8: shows a tool holder for a modular tool in accordance with an embodiment in a third configuration;
- Figure 9: shows a tool holder for a modular tool in accordance with a further embodiment;
- Figure 10: shows a tool in accordance with an embodiment;
- Figure 11a: shows a side view of tool holder for a modular tool substantially as described above with reference to figure 6;
- Figure 11b: shows a side view of tool holder for a modular tool in a variant of the arrangement of figure 11a; and
- Figure 11c: shows a side view of tool holder for a modular tool in a variant of the arrangement of figure 11a.

### Detailed description

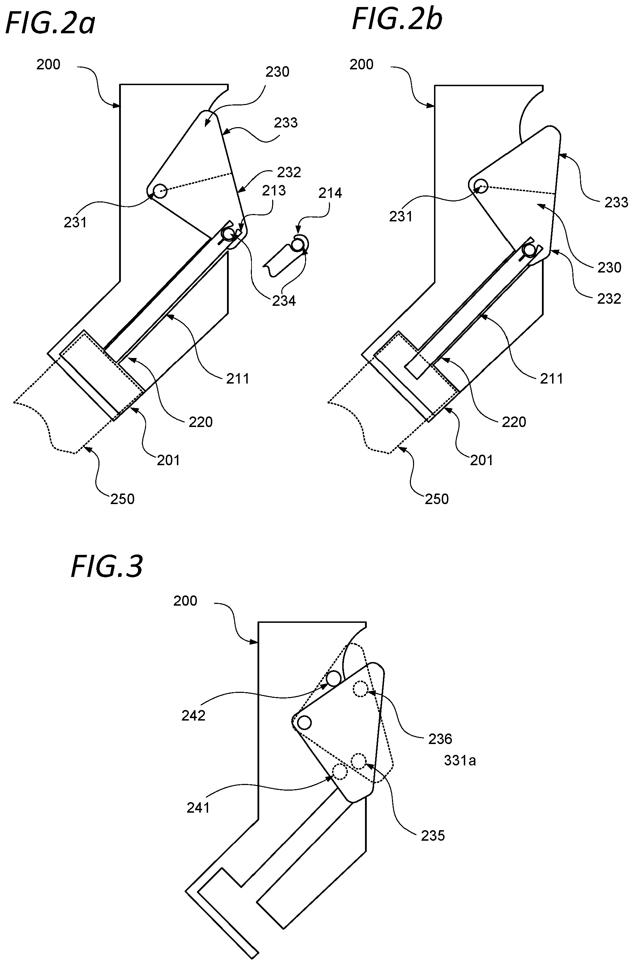

Figure 2a shows a side view of a modular tool in accordance with an embodiment in a first configuration.

As shown in figure 2a there is provided a modular tool comprising a tool holder 200. The tool holder 200 is provided with an entry aperture 201, and adapted to receive a proximal extremity of a working part 250 in the entry aperture. The modular tool further comprises a securing pin assembly comprising a securing pin 211. The tool holder further comprises a securing pin channel 220 extending though the tool holder and terminating in the entry aperture. The securing pin 211 is situated in the securing channel 220 so as to slidingly move between a first, retracted position as shown in the first configuration presented in figure 2a, in which the proximal extremity of the working part 250 is unconstrained by said securing pin in the first position and a second, extended position in which said securing pin protrudes into said entry aperture.

The tool may comprise a surgical instrument. More particularly for example, the tool may be for surgery of the orthopaedic surgery or bone traumatology. Still more particularly for example the tool may comprise a rasp or reamer or impactor.

Figure 2b shows a side view of a modular tool in accordance with the embodiment of figure 2a in a second configuration.

The modular tool of figure 2b comprises the same elements as described above with reference to figure 2a, with like elements being provided with like reference symbols.

As shown in figure 2b, the securing pin 211 is situated in the securing channel 220 in a second, extended position in which said securing pin protrudes into said entry aperture. As shown, in the second position the securing pin 211 traps the proximal extremity of the working part 250 in said entry aperture.

As shown in figures 2a and 2b, the modular tool further comprises a rocker element 230 having a fulcrum 231 pivotally coupled to the tool holder 200, a first arm 232 on one side of the fulcrum pivotally coupled to the securing pin 211, and a second arm 233 on the opposite side of said fulcrum 231 to the first arm. By this means, as shown, pressure on the first arm biases said securing pin towards the second position (securing pin trapping the working part), and pressure on said second arm biases said securing pin towards said first position (working part unconstrained).

In alternate embodiments, e.g. placing the rocker on the opposite side of the tool holder with respect to the angle of operation of the securing pin, a configuration may be achieved whereby a pressure on said first arm biases said securing pin towards said second position, and pressure on said second arm biases said securing pin towards said first position.

As shown in figures 2a and 2b the first arm is pivotally coupled to the securing pin by means of a fork 213 disposed on said securing pin so as to releasable engage a pivot pin 234 disposed in said rocker parallel the axis of said fulcrum. This releasable operation may be achieved by forming said securing pin, or the fork part thereof, of a suitable resilient material such as spring steel, suitably formed plastic or the like. Alternatively, the fork may be formed of a substantially rigid material, and provided with a suitable articulated, spring loaded mechanism adapted to releasably engage the axis of the fulcrum. As shown in figure 2a, other forms besides the fork 213 may be envisaged too achieve this, such as hook 214. The hook form has the advantage of averting the risk of the securing pin accidentally releasing the pivot pin under pressure from the rocker as described above. Accordingly, the fork or hook may be formed of a resilient material and so dimensioned as to be resiliently retained on said pivot pin.

While as shown in figure 2 the articulation of the securing pin and the rocker is achieved by means of a hook or fork engaging a pivot pin, the skilled person will recognize that an equivalent effect may be achieved using other mechanisms. For example, the rocker and securing pin may be linked by a flexible member formed of a suitable resilient material so as to effectively transmit a force from the rocker to the securing pin, while allowing the angular relationship of the rocker and the securing pin to change as the rocker moves. Still further, this flexible member may be formed continuously as excrescence of the rocker or the securing pin, for example by providing a thin, flat section at a suitable extremity. Still further, the rocker or the securing pin may be formed as a single element, having an intermediate flexible section. This approach may simplify the fabrication and assembly of the article, reduce the number of individual parts, and improve hygiene by limiting the presence of small interstices.

While as shown in figure 2 the articulation of the rocker and the tool holder is achieved by means of a hook or fork engaging a pivot pin, the skilled person will recognize that an equivalent effect may be achieved using other mechanisms. For example, the rocker and tool holder may be linked by a flexible member formed of a suitable resilient material so as allow the angular relationship of the rocker and the securing pin to change as the rocker moves, while the virtual pivot point remains at substantially a constant distance from the tool holder body. Still further, this flexible member may be formed continuously as an excrescence of the rocker or the toolholder, for example by providing a thin, flat section at a suitable extremity. Still further, the rocker or the securing pin may be formed as a single element, having an intermediate flexible section. This approach may simplify the fabrication and assembly of the article, reduce the number of individual parts, and improve hygiene by limiting the presence of small interstices.

Figure 3 shows a side view of a modular tool in accordance with a further embodiment.

Figure 3 shows a modular tool similar to that of figures 2a and 2b. As shown, the first arm 232, upon which pressure biases said securing pin towards the second position(working part trapped) and the tool holder 200, are provided with a resiliently biased detent 235, 241 respectively such that when sufficient pressure is exerted on the arm 232, the retention mechanism engages to releasably lock the arm in a first predetermined position, and thereby maintain said securing pin in the second position. This detent may take the form of a spring loaded ball bearing or the like, engaging a recess in the opposite member- the recess may be provided on an inside surface of the rocker 230, or on an outer surface of the tool holder as desired, with the cooperating member of the detent mechanism being provided on the other component as the case may be.

Similarly, the second arm, 233, upon which pressure biases said securing pin towards said first position, and said tool holder, are provided with a resiliently biased detent 236, 242 such that when sufficient pressure is exerted on said second arm, said retention mechanism engages to releasably lock said arm in a second predetermined position, and thereby maintain said securing pin in said first position.

It will be appreciated that either or both detent mechanisms may be provided, and that either or both may implement a spring loaded ball bearing based mechanism, or otherwise, for example as described further below.

In particular, either or both resiliently biased detent comprises a depression formed in said tool holder and a corresponding rounded pin on a resilient projection from said rocker.

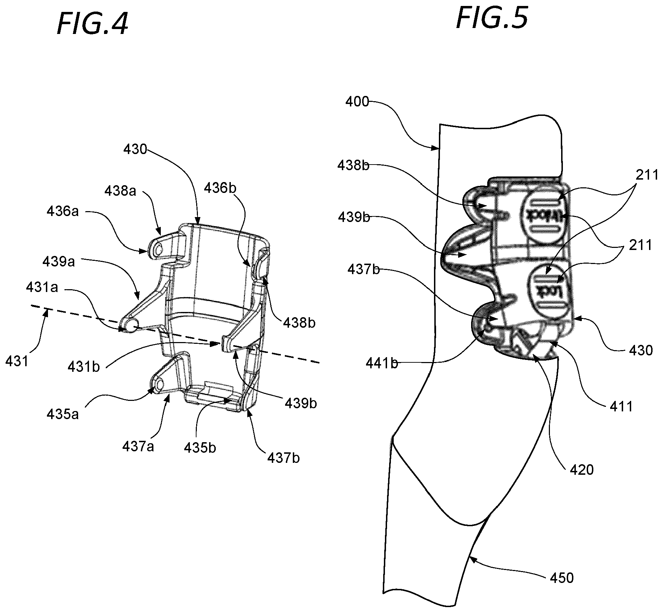

Figure 4 shows a three-dimensional view of a rocker in accordance with an embodiment.

As shown in figure 4, a rocker 430 comprises a lower first rounded pin 435a on a respective lower first resilient projection 437a from one side of said rocker and a corresponding lower second rounded pin 435b on a respective lower second resilient projection 437b from the opposite side of said rocker, so that the lower first and second rounded pins 435a and 435b are biased towards each other when engaged with respective depressions provided in the body of said tool holder, e.g. so as to releasably lock said rocker in said the second position as described above.

Similarly as shown in figure 4, the rocker 430 comprises a first upper rounded pin 436a on a respective first resilient projection 438a from one side of said rocker and a corresponding upper second rounded pin 436b on a respective upper second resilient projection 438b from the opposite side of said rocker, so that the upper first and second rounded pins 436a and 436b are biased towards each other when engaged with respective depressions provided in the body of said tool holder, e.g. so as to releasably lock said rocker in said the first position as described above.

Accordingly, the either or both resiliently biased detents as described above may comprise a first depression formed on one side of the tool holder and a second depression formed on the other side of said tool holder and a corresponding first rounded pin on a respective first resilient projection from one side of said rocker and a corresponding second rounded pin on a respective second resilient projection from the opposite side of said rocker, so that said first and second rounded pins are biased towards each other when engaged with said respective first and second depressions.

Still further, as shown in figure 4, the fulcrum is defined by a further resilient projection 439a, 439b from each side of the rocker, each further resilient projection being 439a, 439b provided with a fulcrum pin 431a, 431b, whereby the fulcrum pin on the further resilient projections 439a, 439b are situated on an axis 431 defining the axis of the fulcrum. The tool holder is provided with apertures engaging said fulcrum pins accordingly, so as to enable the rocker to releasably, and pivotally engage the tool holder.

It will be appreciated that the rocker may be provided with any combination of rounded pins on resilient projections as described with respect to figure 4, and other structures as described herein or as may occur to the skilled person.

It may be noted that the rocker of figure 4 is advantageous in achieving the releasably biased locking mechanisms as well as allowing for the releasable mounting of the rocker on the tool holder, whilst comprising only a single part, which may conveniently be molded, stamped, etc. from a suitable resilient material.

Figure 5 shows the rocker of figure 4 mounted on a tool holder in accordance with an embodiment.

In figure 5 the rocker 430 is visible, mounted on tool holder 400 by means of the engagement between the fulcrum pins (not shown) mounted on resilient projection 439b and 439a (not shown) so as to releasably, and pivotally engage the tool holder as described above. The upper end of the securing pin 411 is visible, emerging from the upper end of the securing pin channel 420, so that as shown the rocker is in the first position as described above, with the securing pin being retracted from the aperture 201, leaving the working part 450 is unconstrained by said securing pin as described above. Accordingly, as shown, the resilient projections 338b (and 438a, not shown) resiliently bias their respective rounded pins 436b (and 436a, not shown) to engage respective depressions (not shown) in the tool holder 420, thereby releasably locking the rocker in the first position.

On this basis, it will be seen that by pressing the lower part of the rocker 430 with sufficient force, the resilient projections 338b (and 438a, not shown) may be brought to disengage their respective resiliently depressions allowing the rocker to pivot about the fulcrum 431 as described above, and to enter the second position as described above. Resilient projections 337b (and 437a, not shown) may then cause their respective rounded pins 435b and 435a, not shown to resiliently bias their respective depression, such as depression 441b in the tool holder 420, thereby releasable locking the rocker in the second position.

The force required to disengage the upper and/or lower resilient projections as described above and move from one position to the other may be defined by the selection of a suitably resilient material, and by specifying the dimensions of the projections, so as to correspond to a force that may be conveniently applied by a user's thumb, without undue effort, but excluding accidental operation.

The force required to disengage the resilient projections of the fulcrum may be defined by the selection of a suitably resilient material, and by specifying the dimensions of the projections, so as to correspond to a force that may exclude decoupling without the deliberate application of significant force, so that once assembled, the tool cannot be accidentally disassembled, even if dropped or brought against another object with substantial force. This may require the use of a dedicated tool or the like.

As shown in figure 5, the rocker may be provided with labels indicated graphically or textually the mode of operation associated with the two positions as described above. In particular, as shown the lower part of the rocker is marked "lock", indicating that by pressing here, the second position is obtained, locking the working part in place, and the upper part of the rocker is marked "unlock" indicating that by pressing here, the first position is obtained, and that the tool may be removed.

As shown, the rocker may be provided with surface texture, ensuring that sufficient force can be safely and effectively brought to bear on the upper and lower portions of the rocker even if the rocker, or the tool as a whole is wet, dirty or otherwise likely to slip under pressure. This texture may comprise grooves as shown, or any other antislip texture as may occur to the skilled person, including the addition of antislip coatings, rubberized secondary material components, and the like. To further facilitate these actions, the elements may be knurled, grooved, provided with a non-slip coating or otherwise treated to improve the users grip thereon. In particular the securing pin may be knurled, grooved, provided with a non-slip coating or provided with a loop, hook, or other formation adapted to engage the users thumb such that the securing pin may be both pushed forward to block the entry aperture, and pulled back to unblock the entry aperture with the users thumb.

As described above, in the second position the securing pin traps the proximal extremity of the tool element in the entry aperture. The skilled person may envisage many possible configuration of the entry aperture, the proximal extremity of the tool element and the securing pin that may achieve this. By way of illustrative example, certain such configurations will not be presented.

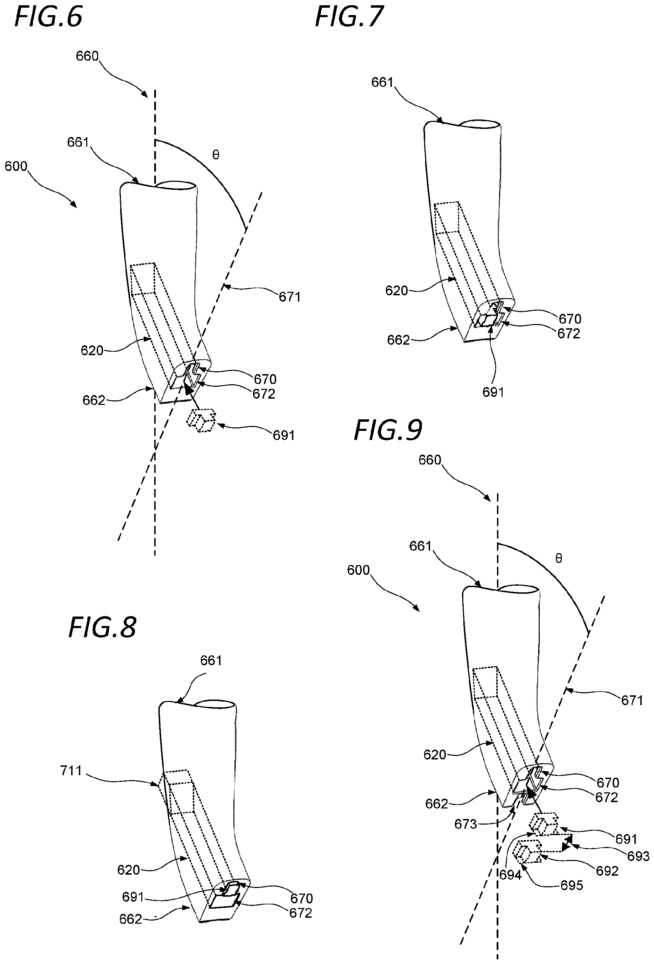

Figure 6 shows a tool holder for a modular tool in accordance with an embodiment in a first configuration.

It may be noted that the rocker, securing pin and other components as described above have been omitted from this figure for the sake of clarity.

As shown, the tool holder 600 has a distal end 662 and a proximal end 661 disposed on a longitudinal axis 660. The tool holder comprises a first lateral slot 670 disposed along a second axis 671, the second axis in the plane of the first axis 660 and being at an angle θ of between 10 and 80 degrees to the longitudinal axis 660. The slot widens from top to bottom, and further widens to an entry aperture 672 at the distal end of the first slot 670.

The tool holder further comprises a securing pin channel 630 substantially as described above extending though the tool holder and terminating in the entry aperture 672.

As shown in figure 6, a working part is provided with a keyed element 691. The keyed element 691 is dimensioned that it may be inserted into the entry aperture 672. Other regions of the working part are excluded for clarity.

As shown in figure 6, the slot 670 defines substantially a T cross section, and the keyed element 691 is shaped correspondingly. This shape achieves the objective of ensuring that the keyed element can only enter or exit the slot through the entry aperture 672, and that once it is slid from the entry aperture to fully engage the slot 670, no force in any direction on the working element with respect to the tool holder will separate the working element secondary element from the tool holder. The skilled person will recognise that the keyed element and corresponding slot may have any form complying with the general requirement that it widens from the distal end towards the proximal end. As such, it may form a wedge, dovetail or T section as described above. It may furthermore be circular, elliptical, rectangular, square, or any other form. The keyed element will generally constitute an extrusion of the chosen cross section from one side to the other. In certain embodiments, the keyed element may taper from one side to the other. Where this is the case the slot may taper from side to side correspondingly. Where this is the case, the slot may be defined as being deeper from side to side that the length from side to side of the corresponding keyed element. On this basis, the tapering walls of the keyed element will engage the sides of the keyed element before the end of the keyed element reached the lateral extremity of the slot. By this means, the slot will become progressively tighter as the keyed element is inserted, and a firm insertion without any play between the handle and secondary element may be achieved by pushing the keyed element fully into the slot.

The disposition of the first lateral slot 670 disposed along a second axis 671, the second axis in the plane of the first axis 660 and being at an angle θ of between 10 and 80 degrees to the longitudinal axis 660 means that the application of a force along the tool holder through the working element will force the working element more deeply in to the slot to abut the end wall thereof, rather than tending to push the working element out of the slot.

Figure 7 shows a tool holder for a modular tool in accordance with an embodiment in a second configuration.

The tool holder of figure 7 comprises substantially the same elements as described with reference to figure 6. As shown in figure 7, the keyed element 691 has been inserted into the entry aperture 672. Other regions of the working part are excluded for clarity.

Figure 8 shows a tool holder for a modular tool in accordance with an embodiment in a third configuration.

The tool holder of figure 8 comprises substantially the same elements as described with reference to figures 6 and 7. As shown in figure 8, the keyed element 691 has been slid upward into the proximal extremity of the slot 670, away from the entry aperture 672. Since the keyed element 691 as shown widens from its base in correspondence to the widening of the slot 670, once slid upward in this manner the keyed element is trapped in the slot, the only possible path of exit being back through the entry aperture 672.

Optionally, the dimensions of the lateral slot may vary along the second axis so that the force required sliding a corresponding keyed element into the lateral slot increases as the corresponding keyed element of the tool progresses into the slot.

As shown in figure 8 the securing pin 711 has been moved through the pin channel 620 by operation of the rocker as described with reference to figure 2a, 2b, 3, 4 or 5 for example, so as to fill the entry aperture 672, thereby trapping the keyed element in the slot 670.

Figure 9 shows a tool holder for a modular tool in accordance with a further embodiment.

The tool holder of figure 9 comprises substantially the same elements as described with reference to figures 6, 7 and 8 above. As shown in figure 9, the tool holder 600 comprises a second slot 673 parallel to the first slot 670. Where this configuration is adopted, it may be considered that the first and second slots define a single continuous slot, which is interrupted by an intermediate wall, and an end wall at the proximal extremity. The second slot 673 may widen from top to bottom in a manner similar to the first slot 670. The second slot 673 may widen from top to bottom identically to the first slot 670. As shown, while the first slot widens to an entry aperture 672 at the distal end of the first slot 670, the second slot 673 opens laterally at the distal extremity of the tool holder 600. As shown, the second slot is on the same axis 671 as the first slot. In other embodiments, the second slot may be situated in a further axis in parallel with the axis 671. Where the second slot does not open at the edge of the tool holder as shown, it may open to a further respective entry aperture in the same way as the first slot. It will be appreciated that any number of slots, and corresponding keyed elements may be provided on this basis. Distributing slots across the distal surface of the tool holder provides multiple connection points with the tool, and as such will tend to stabilize the connection between the working part and tool holder.

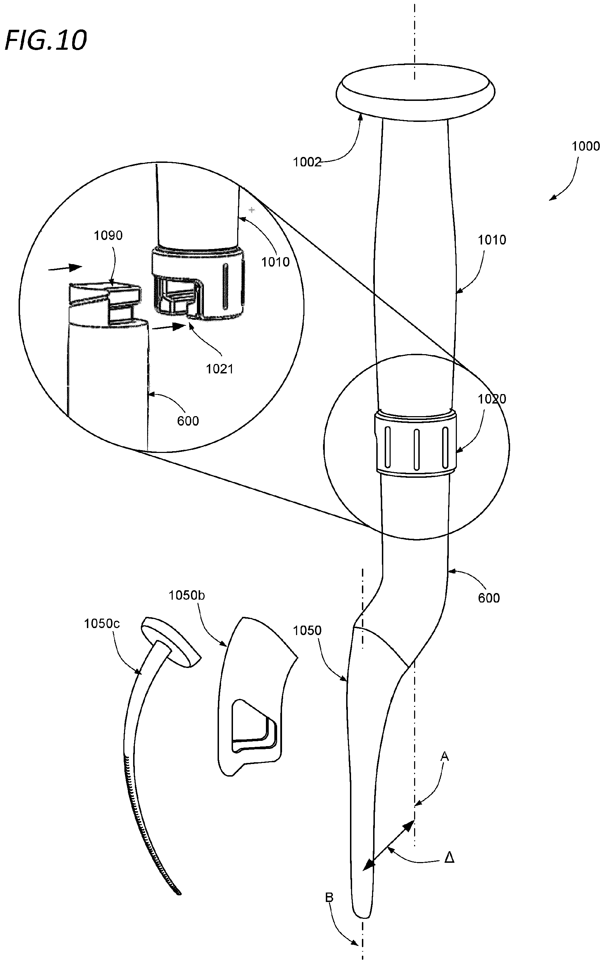

Figure 10 shows a tool in accordance with an embodiment.

As shown, there is provided a modular tool 1000 comprising a handle 1010, and a tool holder 600 substantially as described above. The handle 1010 comprises a releasable coupling comprising by way of example a threaded ring 1020, the threads of the ring engaging an external helical thread of the cylindrical member of the handle 1010. The threaded ring 1020 is rotatable about the distal end of the member between an extended position which the ring obstructs a slot 1021 opening on one periphery of the distal end of the body, and a retracted position as shown in the emphasised section 1050 in which the ring leaves the slot 1021 opening on one periphery of the distal end of the body unobstructed, into which a tongue element 1090 at the proximal extremity of the tool holder may be inserted. When the ring is rotated about the cylindrical member, it progresses along the length thereof and whilst progressing lengthwise and rotating, obstructs a slot 1021 to trap the tool holder in place.

The handle may optionally be provided with an angle datum such as a radial line on the guard plate, or a radial lumen through which a bar may by inserted.

As such the tool holder may further comprise a tongue element provided at a proximal end thereof, the tongue being widening from its junction with the tool holder.

The tool holder may be associated with a handle at the proximal end of the tool holder, and a tool at the distal end of the tool holder releasably coupled thereto by means of a tongue of the tool engaged in the first slot and trapped by the securing pin, where the handle 1010 has a primary axis A and the tool 1000 has a primary axis B, whereby the tool holder 1000 is configured to establish a lateral offset Δ between the two primary axes.

The tool holder may additionally or alternatively establish an angular offset between the axes.

Alternatively a Hudson fitting as known in the state of the art, or any other convenient mechanism may be used to secure the tool holder to the handle. The handle may also be integral to the tool holder.

Meanwhile, the tool holder comprises at a distal end a first lateral slot disposed along a second axis, the second axis in the plane of the first axis and being at an angle of between 10 and 80 degrees to the longitudinal axis, the slot widening from top to bottom, the first slot widening to an entry aperture at the distal end of the first slot, the tool holder further comprising a securing pin channel extending though the tool holder and terminating in the entry aperture, such that a securing pin may be inserted through the pin channel so as to trap a keyed element inserted in the widened part of the first slot, substantially as described above, to provide a releasable coupling between the tool holder 600 and working part 1050. Any of the other variants or optional features presented above may be adopted, or not, in a modular tool along the lines of that of figure 10, as appropriate to the use case.

As shown, the handle further comprises an optional guard plate 1002 at the proximal end thereof. Such a guard plate may serve to protect the hand of a user when gripping the handle 1020 from blows struck against the proximal end thereof with a hammer, mallet or the like, for example where the tool or working part 1050 is a chisel, reamer or other such tool requiring a percussive application.

One field in which a handle as described may be appropriate is that of surgical instruments, such that the modular tool as a whole may comprise or constitutes a surgical instrument. More particularly, the modular tool may be for orthopaedic surgery or bone traumatology. More particularly, the modular tool may be for surgery of the hip, shoulder or knee. More particularly, as shown, the working part 1050, and thus the modular tool as a whole 1050 comprises a rasp or reamer or impactor. It will be appreciated that in line with the many fields of application and associated tool types that may be envisaged, many different possible working parts 1050 may be envisaged, for use with a single handle in accordance with embodiments as described above. Further examples of possible working parts, and resulting modular tools, include a curved rasp 1050b, osteotome 1050c and many other tools as will readily occur to the skilled person.

The tool holder of the present invention may be formed of any material. In particular, it may be formed of steel, aluminium, titanium or any other suitable metal or alloy. It may also be formed of a thermoplastic or other synthetic material. It may in particular be formed from a polyamide, for example a polyarylamide. The synthetic material may comprise additional components such as a filler, swelling agent and the like. It may still further be formed of a synthetic composite material, comprising a glass, carbon fibre, carbon nanoparticle or any other material exhibiting a high tensile strength, in a matrix of a synthetic material, such as any of those listed above. In certain embodiments, the tool holder may be composed of a glass fibre reinforced polyarylamide, such as for example that marketed by the Solvay corporation under the trademark "Ixef GS 1022".

The tool holder may be formed of different materials in different regions, including metal parts and synthetic parts. The handle may also comprise voids for the purpose of economy of material, reduced weight and so on.

Where the tool holder is incorporated in a modular tool as shown in figure 9, the working part and/or handle may each be composed of the materials mentioned above. In some embodiments, the handle, tool holder and working part may all be composed of the same material.

In certain embodiments, the angle of insertion of the working part in the tool holder may be envisaged.

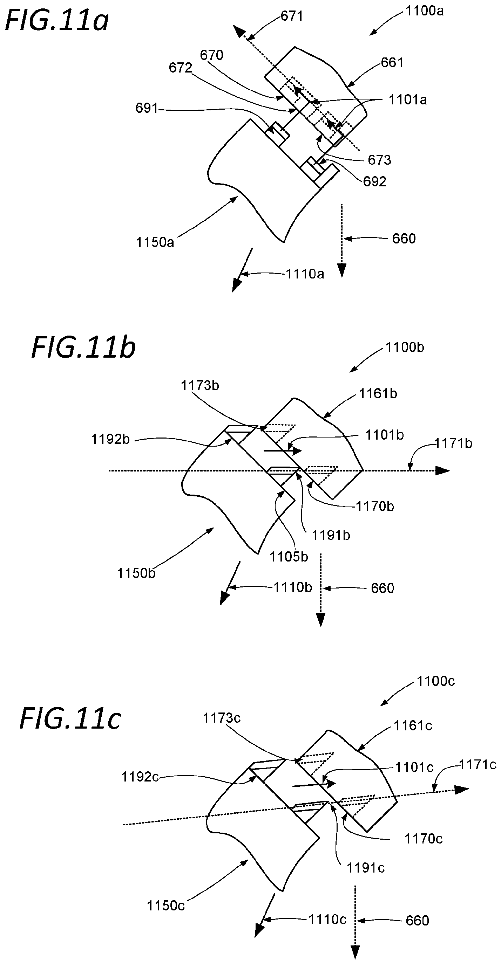

Figure 11a shows a side view of tool holder for a modular tool substantially as described above with reference to figure 6.

In particular, the tool holder 1100a comprises a first lateral slot 670 disposed along a second axis 671, the second axis in the plane of the first axis 670 and being at an angle θ of approximately 45 degrees to the longitudinal axis 670. The slot widens from top to bottom, and further widens to an entry aperture 672 at the distal end of the first slot 670. The working part is provided with a keyed element 691. The keyed element 691 is dimensioned that it may be inserted into the entry aperture 672.

The "push and slide" insertion action implied by the arrangement of figure 11a is represented by arrows 1101a.

As discussed above, the disposition of the first lateral slot 670 disposed along a second axis 671, the second axis in the plane of the first axis 660 and being at an angle θ of between 10 and 80 degrees to the longitudinal axis 660 means that the application of a force along the tool holder through the working element will force the working element more deeply in to the slot to abut the end wall thereof, rather than tending to push the working element out of the slot.

While as discussed with respect to figure 6 the axis 660 is presented as being aligned with the axis of a notional handle which may be provided as discussed herein, it will be appreciated that depending on the shape of the tool holder on one hand, and the tool on the other, a working longitudinal axis 1110a may diverge from the axis of the handle as shown in figure 11a,and that is with respect to this axis that an angle θ of between 10 and 80 degrees may be established to achieve the benefit whereby the application of a force along the tool holder through the working element will force the working element more deeply in to the slot to abut the end wall thereof, rather than tending to push the working element out of the slot.

Figure 11b shows a side view of tool holder for a modular tool in a variant of the arrangement of figure 11a.

In particular, the toolholder 1100b (corresponding substantially to toolholder 600 as described above) comprises a first lateral slot 1170b corresponding substantially to first lateral slot 670 as described above) disposed along a second axis 1171b (corresponding substantially to second axis 671 as described above), the second axis in the plane of the first axis 660 and being at an angle θ of approximately 70 degrees to the longitudinal axis 1160b. The working part is provided with a keyed element 1191b.

The insertion action implied by the arrangement of figure 11b is represented by arrows 1101b.

It may be noted that in the embodiment of figure 11b the keyed elements 1191b and 1192b corresponding to elements 691 and 692 as described above are formed with their respective keyed parts at an angle to the end surface of the tool holder 1105b so that as represented by arrow 1101b the keyed elements may be slid directly into their corresponding slots in a single linear motion.

Figure 11c shows a side view of tool holder for a modular tool in a variant of the arrangement of figure 11a.

In particular, the tool holder 1100c (corresponding substantially to toolholder 600 as described above) comprises a first lateral slot 1070c corresponding substantially to first lateral slot 670 as described above) disposed along a second axis 1171c (corresponding substantially to second axis 671 as described above), the second axis in the plane of the first axis 660 and being at an angle θ of approximately 45 degrees to the longitudinal axis 1110c (and 80 degrees to the longitudinal axis of the notional handle 660). The working part is provided with a keyed element 1191.

The insertion action implied by the arrangement of figure 11c is represented by arrow 1101c.

It may be noted that in the embodiment of figure 11b the keyed elements 1191b and 1192b corresponding to elements 691 and 692 as described above are formed with their respective keyed parts at an angle to the end surface of the tool holder 1105b so that as represented by arrow 1101b the keyed elements may be slid directly into their corresponding slots in a single linear motion.

As such, there is provided a tool holder for a modular tool in which the tool holder may be releasably coupled to a working part such as a rasp, reamer or impactor. The coupling comprises a slot closed at both extremities, and widening to an entrance aperture at one extremity, into which a keyed element of the tool may be inserted, and slid to the opposite extremity of the slot. The modular tool defines a securing pin channel through its body, terminating at the entry aperture such that a securing pin inserted into the channel block the entry aperture and locks the keyed element of the tool in place once inserted.

Accordingly there is provided a tool holder for a modular tool is arranged to receive a tool element which may be inserted laterally by sliding a dovetailed part of the tool into a slot on the tool holder. A securing pin may be inserted through the body of the tool holder so as to enter the slot, thereby locking the tool element in place. The locking pin is pivotally mounted to one side of a rocker, the rocker being pivotally coupled to the tool holder body so that by cycling the rocker from one position to another, the locking pin is moved back and forth through the body of the tool holder to engage or disengage the tool element. The rocker may be of a resilient material so as to clip into position on the tool holder. It may be provided with features such as protrusions adapted to resiliently engage corresponding features on the tool holder so as to latch in one position, or the other, or both.

It will be understood that the configurations described herein are exemplary in nature, and that these specific embodiments or examples are not to be considered in a limiting sense, because numerous variations are possible. The scope of the invention is defined by the appended claims.

## Claims

1. A modular tool comprising a tool holder, said tool holder being provided with an entry aperture (201), and adapted to receive a proximal extremity of a tool element (250) in said entry aperture, said modular tool further comprising a securing pin assembly comprising a securing pin (211), said tool holder further comprising a securing pin channel (220) extending though said tool holder and terminating in said entry aperture, said securing pin (211) being situated in said securing channel (220) so as to slidingly move between a first, retracted position and a second, extended position in which said securing pin protrudes into said entry aperture, whereby in said second position said securing pin traps said proximal extremity of said tool element in said entry aperture, said proximal extremity being unconstrained by said securing pin in said first position, said modular tool further comprising a rocker element (230, 430) having a fulcrum (231) pivotally coupled to said tool holder, a first arm (232) on one side of said fulcrum pivotally coupled to said securing pin (211), and a second arm (233) on the opposite side of said fulcrum to said first arm (232), such that either
a. pressure brought to bear by a user's thumb on said first arm (232) biases said securing pin (211) towards said first position, and pressure brought to bear by a user's thumb on said second arm (233) biases said securing pin towards said second position, or
b. pressure brought to bear by a user's thumb on said first arm (232) biases said securing pin towards said second position, and pressure brought to bear by a user's thumb on said second arm (233) biases said securing pin towards said first position.

2. The modular tool of claim 1, wherein the said arm upon which pressure biases said securing pin towards said first position, and said tool holder, are provided with a resiliently biased detent (438b) such that when sufficient pressure is exerted on said arm, said retention mechanism engages to releasably lock said arm in a first predetermined position, and thereby maintain said securing pin in said first position.

3. The modular tool of claim 1 or 2, wherein the said arm upon which pressure biases said securing pin towards said second position, and said tool holder, are provided with a resiliently biased detent (437b, 441b) such that when sufficient pressure is exerted on said arm, said retention mechanism engages to releasably lock said arm in a second predetermined position, and thereby maintain said securing pin in said second position.

4. The modular tool of claim 2 or 3, wherein said resiliently biased detent comprises a depression (441b) formed in said tool holder and a corresponding rounded pin on a resilient projection (437b) from said rocker (230, 430).

5. The modular tool of claim 4, wherein said resiliently biased detent comprises a first depression (441b) formed on one side of said tool holder and a second depression formed on one side of said tool holder and a corresponding first rounded pin (437b) on a respective first resilient projection from one side of said rocker (230, 430) and a corresponding second rounded pin on a respective second resilient projection from the opposite side of said rocker, so that said first and second rounded pins are biased towards each other when engaged with said respective first and second depressions.

6. The modular tool of any preceding claim, wherein said fulcrum is defined by a further resilient projection (439a, 439b) from each side of said rocker (230, 430), each said further resilient projection being provided with a fulcrum pin (431a, 431b), whereby the fulcrum pin on each further resilient projection is situated on an axis defining the axis of said fulcrum (431), said tool holder being provided with apertures engaging said fulcrum pins.

7. The modular tool of any preceding claim, said first arm is pivotally coupled to said securing pin by means of a fork (213) or hook (214) disposed on said securing pin (211) releasably engaging a pivot pin (234) disposed in said rocker parallel the axis of said fulcrum (231).

8. The modular tool of claim 7, said fork (213) or hook (214) being formed of a resilient material and so dimensions as to be resiliently retained on said pivot pin (234).

9. The modular tool of any preceding claim, said tool holder having a distal end (662) and a proximal end (661) disposed on a longitudinal axis (660), wherein said entry aperture (672) comprises a first lateral slot disposed along a second axis (671), said second axis in the plane of the first axis and being at an angle of between 10 and 80 degrees to said longitudinal axis, said slot widening from top to bottom, said first slot widening to an entry aperture (670) at the distal end of the first slot, said a securing pin channel extending though said tool holder.

10. The modular tool of claim 9 wherein the dimensions of said lateral slot vary along said second axis so that the force required to slide a corresponding keyed element of said tool into said lateral slot increases as said corresponding keyed element of said tool progresses into said slot.

11. The modular tool of any preceding claim comprising a second slot parallel to the first slot.

12. The modular tool of claim 11 wherein said second slot is on the same axis as the first slot, and positioned on the distal side thereof.

13. The modular tool of claim 11 or 12 wherein said second slot provides a lateral opening in said tool holder.

14. The modular tool of any preceding claim wherein said tool holder is composed of a synthetic material or a synthetic composite material.

15. The modular tool of claim 14 wherein said tool holder is composed of a glass fiber reinforced polyarylamide.

## Patentansprüche

1. Modulares Werkzeug, das einen Werkzeughalter umfasst, wobei der Werkzeughalter mit einer Eintrittsöffnung (201) versehen und angepasst ist, um ein proximales Ende eines Werkzeugelements (250) in der Eintrittsöffnung aufzunehmen, wobei das modulare Werkzeug ferner eine Sicherungsstiftbaugruppe umfasst, welche einen Sicherungsstift (211) umfasst, wobei der Werkzeughalter ferner einen Sicherungsstiftkanal (220) umfasst, der sich durch den Werkzeughalter erstreckt und in der Eintrittsöffnung endet, wobei der Sicherungsstift (211) so in dem Sicherungskanal (220) angeordnet ist, dass er sich gleitend zwischen einer ersten, eingezogenen Stellung und einer zweiten, ausgefahrenen Stellung, in welcher der Sicherungsstift in die Eintrittsöffnung vorspringt, bewegt, wodurch der Sicherungsstift in der zweiten Stellung das proximale Ende des Werkzeugelements in der Eintrittsöffnung einfängt, wobei das proximale Ende durch den Sicherungsstift in der ersten Stellung nicht eingeschränkt wird, wobei das modulare Werkzeug ferner ein Kipphebelelement (230, 430) umfasst, das einen Drehpunkt (231), der schwenkbar mit dem Werkzeughalter verbunden ist, einen ersten Arm (232) auf der einen Seite des Drehpunktes, der schwenkbar mit dem Sicherungsstift (211) verbunden ist, und einen zweiten Arm (233) auf der zu dem ersten Arm (232) entgegengesetzten Seite des Drehpunktes aufweist, so dass entweder
a. Druck, der durch den Daumen eines Benutzers auf den ersten Arm (232) angewendet wird, den Sicherungsstift (211) hin zu der ersten Stellung vorspannt und Druck, der durch den Daumen eines Benutzers auf den zweiten Arm (233) angewendet wird, den Sicherungsstift hin zu der zweiten Stellung vorspannt, oder
b. Druck, der durch den Daumen eines Benutzers auf den ersten Arm (232) angewendet wird, den Sicherungsstift hin zu der zweiten Stellung vorspannt und Druck, der durch den Daumen eines Benutzers auf den zweiten Arm (233) angewendet wird, den Sicherungsstift hin zu der ersten Stellung vorspannt.

2. Modulares Werkzeug nach Anspruch 1, wobei der Arm, auf den Druck den Sicherungsstift hin zu der ersten Stellung vorspannt, und der Werkzeughalter mit einer elastisch vorgespannten Rastvorrichtung (438b) versehen sind, so dass, wenn ausreichender Druck auf den Arm ausgeübt wird, der Rückhaltemechanismus einrückt, um den Arm in einer ersten vorbestimmten Stellung zu arretieren und dadurch den Sicherungsstift in der ersten Stellung zu halten.

3. Modulares Werkzeug nach Anspruch 1 oder 2, wobei der Arm, auf den Druck den Sicherungsstift hin zu der zweiten Stellung vorspannt, und der Werkzeughalter mit einer elastisch vorgespannten Rastvorrichtung (437b, 441b) versehen sind, so dass, wenn ausreichender Druck auf den Arm ausgeübt wird, der Rückhaltemechanismus einrückt, um den Arm in einer zweiten vorbestimmten Stellung lösbar zu arretieren und dadurch den Sicherungsstift in der zweiten Stellung zu halten.

4. Modulares Werkzeug nach Anspruch 2 oder 3, wobei die elastisch vorgespannte Rastvorrichtung eine Vertiefung (441b), die in dem Werkzeughalter geformt ist, und einen entsprechenden abgerundeten Stift an einem elastischen Vorsprung (437b) von dem Kipphebel (230, 430) umfasst.

5. Modulares Werkzeug nach Anspruch 4, wobei die elastisch vorgespannte Rastvorrichtung eine erste Vertiefung (441b), die auf einer Seite des Werkzeughalters geformt ist, und eine zweite Vertiefung, die auf einer Seite des Werkzeughalters geformt ist, und einen entsprechenden ersten abgerundeten Stift (437b) an einem jeweiligen ersten elastischen Vorsprung von der einen Seite des Kipphebels (230, 430) und einen entsprechenden zweiten abgerundeten Stift an einem jeweiligen zweiten elastischen Vorsprung von der entgegengesetzten Seite des Kipphebels umfasst, so dass der erste und der zweite abgerundete Stift zueinander hin vorgespannt werden, wenn sie mit der jeweiligen ersten beziehungsweise zweiten Vertiefung in Eingriff gebracht werden.

6. Modulares Werkzeug nach einem der vorhergehenden Ansprüche, wobei der Drehpunkt durch einen weiteren elastischen Vorsprung (439a, 439b) von jeder Seite des Kipphebels (230, 430) definiert wird, wobei jeder weitere elastische Vorsprung mit einem Drehbolzen (431a, 431b) versehen ist, wobei der Drehbolzen an jedem weiteren elastischen Vorsprung auf einer Achse angeordnet ist, welche die Achse des Drehpunktes (431) definiert, wobei der Werkzeughalter mit Öffnungen versehen ist, welche die Drehbolzen in Eingriff nehmen.

7. Modulares Werkzeug nach einem der vorhergehenden Ansprüche, wobei der erste Arm schwenkbar mit dem Sicherungsstift verbunden ist mit Hilfe einer Gabel (213) oder eines Hakens (214), angeordnet an dem Sicherungsstift (211), die/der lösbar einen Drehzapfen (234) in Eingriff nimmt, der in dem Kipphebel parallel zu der Achse des Drehpunktes (231) angeordnet ist.

8. Modulares Werkzeug nach Anspruch 7, wobei die Gabel (213) oder der Haken (214) aus einem elastischen Material geformt und so bemessen ist, dass sie/er elastisch an dem Drehzapfen (234) festgehalten wird.

9. Modulares Werkzeug nach einem der vorhergehenden Ansprüche, wobei der Werkzeughalter ein distales Ende (662) und ein proximales Ende (661), die auf einer Längsachse (660) angeordnet sind, aufweist, wobei die Eintrittsöffnung (672) einen ersten seitlichen Schlitz umfasst, der entlang einer zweiten Achse (671) angeordnet ist, wobei die zweite Achse sich in der Ebene der ersten Achse befindet und in einem Winkel von zwischen 10 und 80 Grad zu der Längsachse liegt, wobei sich der Schlitz von oben nach unten weitet, wobei sich der erste Schlitz zu einer Eintrittsöffnung (670) an dem distalen Ende des ersten Schlitzes weitet, wobei sich ein Sicherungsstiftkanal durch den Werkzeughalter erstreckt.

10. Modulares Werkzeug nach Anspruch 9, wobei sich die Abmessungen des seitlichen Schlitzes entlang der zweiten Achse so verändern, dass die Kraft, die erforderlich ist, um ein entsprechendes verkeiltes Element des Werkzeugs in den seitlichen Schlitz zu schieben, zunimmt, wenn das entsprechende verkeilte Element des Werkzeugs in den Schlitz voranschreitet.

11. Modulares Werkzeug nach einem der vorhergehenden Ansprüche, das einen zweiten Schlitz parallel zu dem ersten Schlitz umfasst.

12. Modulares Werkzeug nach Anspruch 11, wobei sich der zweite Schlitz auf derselben Achse befindet wie der erste Schlitz und auf der distalen Seite davon angeordnet ist.

13. Modulares Werkzeug nach Anspruch 11 oder 12, wobei der zweite Schlitz eine seitliche Öffnung in dem Werkzeughalter bereitstellt.

14. Modulares Werkzeug nach einem der vorhergehenden Ansprüche, wobei der Werkzeughalter aus einem synthetischen Werkstoff oder einem synthetischen Verbundwerkstoff besteht.

15. Modulares Werkzeug nach Anspruch 14, wobei der Werkzeughalter aus einem glasfaserverstärkten Polyarylamid besteht.

## Revendications

1. Outil modulaire comprenant un porte-outil, ledit porte-outil étant doté d'une ouverture d'entrée (201) et adapté pour recevoir une extrémité proximale d'un élément d'outil (250) dans ladite ouverture d'entrée, ledit outil modulaire comprenant en outre un ensemble de broche de fixation comprenant une broche de fixation (211), ledit porte-outil comprenant en outre un canal de broche de fixation (220) s'étendant à travers ledit porte-outil et se terminant dans ladite ouverture d'entrée, ladite broche de fixation (211) étant située dans ledit canal de fixation (220) de façon à se déplacer en coulissant entre une première position rétractée et une seconde position déployée, dans laquelle ladite broche de fixation ressort dans ladite ouverture d'entrée, moyennant quoi dans ladite seconde position, ladite broche de fixation piège ladite extrémité proximale dudit élément d'outil dans ladite ouverture d'entrée, ladite extrémité proximale n'étant pas contrainte par ladite broche de fixation dans ladite première position, ledit outil modulaire comprenant en outre un élément culbuteur (230, 430) présentant un pivot (231) couplé en pivotement audit porte-outil, un premier bras (232) d'un côté dudit pivot couplé en pivotement à ladite broche de fixation (211), et un second bras (233) sur le côté opposé dudit pivot audit premier bras (232), de sorte que soit
a. une pression exercée par le pouce d'un utilisateur sur ledit premier bras (232) polarise ladite broche de fixation (211) vers ladite première position, et la pression exercée par le pouce d'un utilisateur sur ledit second bras (233) polarise ladite broche de fixation vers ladite seconde position, ou
b. une pression exercée par le pouce d'un utilisateur sur ledit premier bras (232) polarise ladite broche de fixation vers ladite seconde position, et la pression exercée par le pouce d'un utilisateur sur ledit second bras (233) polarise ladite broche de fixation vers ladite première position.

2. Outil modulaire selon la revendication 1, dans lequel ledit bras sur lequel une pression polarise ladite broche de fixation vers ladite première position, et ledit porte-outil, sont dotés d'un cran polarisé de manière résiliente (438b), de sorte que lorsqu'une pression suffisante est exercée sur ledit bras, ledit mécanisme de retenue se met en prise pour verrouiller de manière détachable ledit bras dans une première position prédéterminée, et maintenir ainsi ladite broche de fixation dans ladite première position.

3. Outil modulaire selon la revendication 1 ou 2, dans lequel ledit bras sur lequel une pression polarise ladite broche de fixation vers ladite seconde position, et ledit porte-outil, sont dotés d'un cran polarisé de manière résiliente (437b, 441b) de sorte que, lorsqu'une pression suffisante est exercée sur ledit bras, ledit mécanisme de retenue se met en prise pour verrouiller de manière détachable ledit bras dans une seconde position prédéterminée, et maintenir ainsi ladite broche de fixation dans ladite seconde position.

4. Outil modulaire selon la revendication 2 ou 3, dans lequel ledit cran polarisé de manière résiliente comprend un creux (441b) formé dans ledit porte-outil et une broche arrondie correspondante sur une projection résiliente (437b) à partir dudit culbuteur (230, 430).

5. Outil modulaire selon la revendication 4, dans lequel ledit cran polarisé de manière résiliente comprend un premier creux (441b) formé sur un côté dudit porte-outil et un second creux formé sur un côté dudit porte-outil et une première broche arrondie correspondante (437b) sur une première projection résiliente respective depuis un côté dudit culbuteur (230, 430) et une seconde broche arrondie correspondante sur une seconde projection résiliente respective depuis le côté opposé dudit culbuteur, de sorte que lesdites première et seconde broches arrondies sont polarisées les unes vers les autres, lorsqu'elles sont mises en prise avec lesdits premier et second creux respectifs.

6. Outil modulaire selon l'une quelconque des revendications précédentes, dans lequel ledit pivot est défini par une projection résiliente supplémentaire (439a, 439b) depuis chaque côté dudit culbuteur (230, 430), chaque dite projection résiliente supplémentaire étant dotée d'une broche pivot (431a, 431b), dans lequel la broche pivot sur chaque projection résiliente supplémentaire est située sur un axe définissant l'axe dudit pivot (431), ledit porte-outil étant doté d'ouvertures mettant en prise lesdites broches pivots.

7. Outil modulaire selon l'une quelconque des revendications précédentes, ledit premier bras est couplé en pivotement à ladite broche de fixation au moyen d'une fourche (213) ou d'un crochet (214) disposé sur ladite broche de fixation (211) en mettant en prise de manière détachable une broche de pivot (234) disposée dans ledit culbuteur, parallèlement à l'axe dudit pivot (231).

8. Outil modulaire selon la revendication 7, ladite fourche (213) ou ledit crochet (214) étant formé(e) d'un matériau résilient et dimensionné(e) de façon à être maintenu(e) de manière résiliente sur ladite broche de pivot (234).

9. Outil modulaire selon l'une quelconque des revendications précédentes, ledit porte-outil présentant une extrémité distale (662) et une extrémité proximale (661) disposées sur un axe longitudinal (660), dans lequel ladite ouverture d'entrée (672) comprend une première fente latérale disposée le long d'un second axe (671), ledit second axe étant dans le plan du premier axe et étant selon un angle compris entre 10 et 80 degrés par rapport audit axe longitudinal, ladite fente s'élargissant de haut en bas, ladite première fente s'élargissant vers une ouverture d'entrée (670) à l'extrémité distale de la première fente, ledit un canal de broche de fixation s'étendant à travers le porte-outil.

10. Outil modulaire selon la revendication 9, dans lequel les dimensions de ladite fente latérale varient le long dudit second axe de sorte que la force requise pour faire coulisser un élément calé correspondant dudit outil dans ladite fente latérale augmente au fur et à mesure que ledit élément calé correspondant dudit outil progresse dans ladite fente.

11. Outil modulaire selon l'une quelconque des revendications précédentes, comprenant une seconde fente parallèle à la première fente.

12. Outil modulaire selon la revendication 11, dans lequel ladite seconde fente est sur le même axe que la première fente et positionnée sur le côté distal de celle-ci.

13. Outil modulaire selon la revendication 11 ou 12, dans lequel ladite seconde fente fournit une ouverture latérale dans ledit porte-outil.

14. Outil modulaire selon l'une quelconque des revendications précédentes, dans lequel ledit porte-outil est composé d'un matériau synthétique ou d'un matériau composite synthétique.

15. Outil modulaire selon la revendication 14, dans lequel ledit porte-outil est composé d'un polyarylamide renforcé en fibre de verre.
